# EUROPÄISCHE PATENTSCHRIFT

(11) **EP 3 638 354 B1**
(45) Veröffentlichungstag und Bekanntmachung des Hinweises auf die Patenterteilung: **26.04.2023**
(21) Anmeldenummer: 18731746.6
(22) Anmeldetag: 29.05.2018
(51) Int. Cl.: A61M 25/09, A61M 25/01

(54) **FÜHRUNGSDRAHT FÜR MINIMALINVASIVE EINGRIFFE SOWIE VERFAHREN ZUR HERSTELLUNG EINES FÜHRUNGSDRAHTES**
GUIDE WIRE FOR MINIMALLY INVASIVE INTERVENTIONS AND METHOD FOR MANUFACTURING A GUIDE WIRE
FIL DE GUIDAGE POUR INTERVENTIONS À INVASION MINIMALE ET PROCÉDÉ DE PRODUCTION D'UN FIL DE GUIDAGE

(30) Priorität: 16.06.2017 EP 17176412
(43) Veröffentlichungstag der Anmeldung: 22.04.2020
(73) Patentinhaber: Nano4Imaging GmbH, 40225 Düsseldorf (DE)
(72) Erfinder: BORM, Paul J. A., 6231 CV Meerssen (NL); MANEGOLD, Christoph R., 40549 Düsseldorf (DE); CREMERS, Jozef G. O., 6411 LP Heerlen (NL)
(74) Vertreter: Naeven, Ralf
(86) Internationale Anmeldenummer: PCT/EP2018/064118
(87) Internationale Veröffentlichungsnummer: WO 2018/228817

(56) Entgegenhaltungen:
- EP-A1- 2 098 262
- EP-A1- 2 548 604
- DE-A1-102005 022 688
- US-A1- 2004 167 438
- US-A1- 2014 121 648

## Beschreibung

Die Erfindung betrifft einen Führungsdraht gemäß dem Oberbegriff des Anspruchs 1 sowie ein Verfahren zur Herstellung eines Führungsdrahtes gemäß dem Oberbegriff des Anspruchs 11.

Aus der EP 1 348 461 B1 ist ein Führungsdraht offenbart, bei dem ein innerer Schaft des Führungsdrahts aus einem Metall oder einem Kunststoff mit relativ hoher Steifheit von einer Kunststoffschicht umgeben ist. Am distalen Drahtendstück des Führungsdrahtes ist der Kern kegelförmig zugespitzt, um eine höhere Flexibilität des Drahtendstücks zu erreichen.

Die US 2014/0121648 A1 offenbart einen Führungsdraht mit einer Verjüngung am distalen Ende eines Kerns aus einem Verbundmaterial, wobei die Verjüngung die Flexibilität des Drahtendstücks erhöhen soll. Die Formgebung der Kernspitze erfolgt mittels Schleifen oder Schneiden.

Die US 2004/0167438 A1 offenbart ebenfalls einen Führungsdraht mit einer Verjüngung am distalen Ende eines Kerns, wobei die Verjüngung des Kerns in mehreren Stufen vorgesehen sein kann. Auch hier sollen mit der Form der Kernspitze gewünschte mechanische Eigenschaften, z.B. hinsichtlich der Flexibilität des Drahtendstücks erreicht werden.

Die EP 2 098 262 A1 offenbart einen Katheter-Führungsdraht mit einem Kern, in dessen Grundmaterial verdrillte Fasern, vorzugsweise Glasfasern eingebettet sind, und dessen distales Drahtendstück eine durch Schleifen erzeugte, sich verjüngende Form aufweisen kann.

Aus der DE 10 2005 022 688 A1 ist ebenfalls ein Führungsdraht mit einem am distalen Ende verjüngten Kern bekannt.

Je nach Herstellungsverfahren für den Kern eines Führungsdrahtes ist eine Durchmesserverringerung des Kerns, sei es kegelförmig oder in Stufen, aufwendig. Insbesondere Schleifverfahren sind zeit- und kostenintensiv.

Aus der EP 2 548 604 A1 sind ein Führungsdraht und ein Verfahren der eingangs genannten Art bekannt. Der Führungsdraht weist einen sich auch in ein distales Drahtendstück hinein erstreckenden Kern mit zufällig verteilten nicht-metallischen Fasern oder Fasersegmenten und einem die Zwischenräume zwischen den Fasern füllenden Harz auf. In einem Ausführungsbeispiel weist der Kern radial ausgerichtete Schnitte auf seinem Umfang auf, die dazu dienen sollen, das distale Drahtendstück flexibler zu machen. Die Schnitte können durch Schneiden oder Ätzen erzeugt werden. Die Tiefe oder Weite der Schnitte oder der Abstand zwischen den Schnitten kann je nach gewünschter Flexibilität angepasst werden.

Der Erfindung liegt das technische Problem zugrunde, einen Führungsdraht mit einer in einem Drahtendstück gegebenen höheren Flexibilität sowie ein Verfahren zu dessen Herstellung zur Verfügung zu stellen, wobei der Führungsdraht an seinem distalen Drahtendstück einen gegenüber dem Stand der Technik alternativen, vereinfacht herstellbaren Aufbau aufweist.

Bei einem Führungsdraht der eingangs genannten Art wird das technische Problem mit den kennzeichnenden Merkmalen des Anspruchs 1 gelöst. Bei einem Verfahren der eingangs genannten Art wird das technische Problem durch die Merkmale des Anspruchs 11 gelöst. Vorteilhafte Ausführungsformen der Erfindung ergeben sich aus den Unteransprüchen.

Demnach wird vorgeschlagen, dass ein innerer Schaft zumindest in einem distalen Drahtendstück eine Vielzahl von Schwächungsstellen aufweist, welche durch mechanische Eingriffe, nämlich durch Knickbelastung, Biegebelastung und/oder Brechbelastung erzeugt sind.

Die Schwächungsstellen erniedrigen die Biegesteifigkeit des inneren Schaftes und damit des distalen Drahtendstückes, das heißt, dessen Biegemodul wird verringert. Mit der verringerten Biegesteifigkeit erhöht sich die Flexibilität des Führungsdrahtes und dessen Fähigkeit, gekrümmten Wegstrecken zu folgen.

Brechen, Knicken oder Biegen wirkt auf den gesamten Querschnitt des Kernes, ohne dass hierdurch ein völliges Durchtrennen des Kerns bewirkt wird. Insbesondere die feinen Fasern des Faserverbundmaterials werden durch die Biege-, Knick-, oder Brechbewegung zumindest zum Teil nicht durchtrennt und bewirken trotz der Schwächung einen Zusammenhalt des Kerns. Das Brechen, Knicken oder Biegen zum Einbringen der Schwächungsstellen kann zudem deutlich kosten- und zeitsparender gestaltet werden als das aus dem Stand der Technik bekannte Einschneiden oder Einätzen der Schwächungsstellen in den inneren Schaft.

Die Anzahl von Schwächungsstellen hängt insbesondere von der Länge des distalen Drahtendstückes ab. So kann das distale Drahtendstück zum Beispiel 10 mm bis 50 mm betragen. Kürzere oder längere distale Drahtendstücke sind ebenfalls möglich. Vorzugsweise weisen die Schwächungsstellen in axialer Richtung des Führungsdrahtes regelmäßige Abstände voneinander auf.

Die Abstände zwischen den Schwächungsstellen sind bevorzugt im Millimeter-Bereich. Vorzugsweise kann ein distales Drahtendstück über eine Länge von 30 bis 60 mm, vorzugsweise 40 mm, Schwächungsstellen aufweisen, die etwa 1 mm bis 3 mm, z.B. 2 mm +/- 0.5 mm, voneinander beabstandet sind.

Zur Herstellung des distalen Drahtendstückes wird ein Verfahren vorgeschlagen, bei dem der zumindest im distalen Drahtendstück vorgesehene innere Schaft durch mechanische Eingriffe mit einer Vielzahl von Schwächungsstellen versehen wird, wobei die Schwächungsstellen durch Knickbelastung, Biegebelastung und/oder Brechbelastung erzeugt werden.

Das erfindungsgemäße Verfahren kann insbesondere so ausgeführt werden, dass für die mechanische Eingriffe der innere Schaft über mindestens eine mechanische Kante gelegt und mit einer quer zur Längsachse des ungeschwächten Drahtendstücks wirkenden Kraft beaufschlagt wird. So kann der innere Schaft beispielsweise über die mechanische Kante gelegt und die Kraft an einem über die Kante hinausragenden Teil des inneren Schafts angreifen. Für die Erzeugung der folgenden Schwächungsstellen braucht der innere Schaft lediglich um eine geeignete Strecke, z.B. mit einer Streckenlänge von 1mm bis 3 mm, vorgeschoben zu werden. Die Kraft kann die Gewichtskraft einer am inneren Schaft z.B. durch Klemmung fixierten Masse sein.

Das erfindungsgemäße Verfahren kann so ausgeführt werden, dass die mechanischen Eingriffe in mindestens zwei unterschiedlichen Drehwinkelpositionen des inneren Schafts durchgeführt werden. Die Drehwinkelposition bezieht sich auf eine Rotation um die Längsachse des inneren Schaftes. So kann z.B. der innere Schaft, ohne ihn um seine Längsachse zu drehen, an einer Vielzahl von Schwächungsstellen geschwächt werden. Anschließend kann der innere Schaft relativ zu der angreifenden Kraft um einen bestimmten Drehwinkel, z.B. von 90° oder in einem Bereich von 80° bis 100°, um die Längsachse gedreht werden, um anschließend im durch den ersten Vorgang bereits geschwächten Bereich weitere Schwächungsstellen einzubringen oder bereits vorhandene Schwächungsstellen weiter zu schwächen. Auf diese Weise wird sichergestellt, dass die Schwächung die Biegesteifigkeit des inneren Schaftes im Drahtendbereich nicht nur in einer Biegerichtung verringert wird.

Der mechanische Eingriff wird vorzugsweise unmittelbar am inneren Schaft durchgeführt, d.h. ohne Umhüllung des inneren Schaftes mit einer Schutzschicht. Es kann aber auch bereits eine Schutzschicht angebracht sein.

Beim Schwächungsvorgang kann Material aus dem inneren Schaft ausbrechen. Vorzugsweise bleibt jedoch - abgesehen von den genannten eventuellen, regelmäßig kleinkörnigen Ausbrechungen - der Durchmesser des inneren Schafts im geschwächten Bereich zumindest im Mittel weitgehend konstant.

Vorzugsweise weist der innere Schaft des Führungsdrahts ein erstes Faserverbundmaterial auf. Die vorzugsweise von einem Kunststoff-Matrixmaterial umhüllten und/oder miteinander verklebten Fasern des Faserverbundmaterials geben dem Schaft eine geeignete Stabilität. Insbesondere können die Fasern beim mechanischen Schwächen des inneren Schaftes dem inneren Schaft noch hinreichenden Zusammenhalt geben, so dass kein vollständiger Bruch, also keine komplette Teilung des inneren Schafts auftritt.

Alternativ kann beim mechanischen Schwächen ein Auseinanderfallen des inneren Schafts aber auch durch die mindestens eine den inneren Schaft umhüllende Schutzschicht verhindert werden.

Es kann vorteilhaft sein, mit dem erfindungsgemäßen Verfahren den inneren Schaft so auszubilden, dass er einen Kern und mindestens eine den Kern umgebende Hüllschicht aufweist. Dabei kann der Kern das erste Faserverbundmaterial aufweisen oder aus ihm bestehen. Weiter vorteilhaft kann die mindestens eine Hüllschicht oder mindestens eine der Hüllschichten ein zweites Faserverbundmaterial aufweisen, wobei das zweite Faserverbundmaterial bevorzugt vom ersten Faserverbundmaterial verschieden ist.

Mit der Kombination aus Kern und Hüllschicht lassen sich gewünschte mechanische Eigenschaften des inneren Schaftes einstellen. So können die beiden Faserverbundmaterialien in ihren Materialien übereinstimmen oder im Fasermaterial und/oder im die Fasern miteinander verklebenden oder diese umhüllenden Matrixmaterial unterschiedlich sein. Bei dem Matrixmaterial kann es sich um einen Harz, zum Beispiel Kunstharz, insbesondere Epoxidharz, oder einen sonstigen Kunststoff handeln

Vorzugsweise werden die Fasern der Hüllschicht zumindest in einer Teilanzahl helixartig um den Umfang des Kerns geführt. Eine helixartige Führung der Fasern bedeutet, dass diese nicht durchgehend in axialer Richtung verlaufen sondern in Form einer Helix den Kern umgeben. Die helixartige Führung der Fasern sorgt insbesondere außerhalb des mit Schwächungsstellen versehenen Bereichs für eine erhöhte Torsionssteifigkeit, sorgt also für eine verbesserte Übertragbarkeit von Drehmomenten. Die Biegesteifigkeit kann außerhalb des mit Schwächungsstellen versehenen Bereichs im Wesentlichen durch den Kern mit seinen durch das Matrixmaterial des Kerns miteinander verklebten Fasern bestimmt werden.

Im distalen Drahtendstück hingegen kann durch das Einbringen der Schwächungsstellen die Biegesteifigkeit in einem gewünschten Maß verringert werden, wobei das Maß der Verringerung der Biegesteifigkeit durch die in axialer Richtung gegebene Dichte der Schwächungsstellen beeinflusst werden kann.

Vorzugsweise werden die Fasern zumindest in einer Teilanzahl in mindestens zwei unterschiedlichen Helixorientierungen um den Umfang des Kerns geführt, so dass die Torsionssteifigkeit für beide Umfangsrichtungen erhöht ist. Die Fasern der Hüllschicht sind von einem Matrixmaterial umhüllt und/oder mittels des Matrixmaterials miteinander verklebt. Bei dem Matrixmaterial kann es sich um einen Harz, zum Beispiel Kunstharz, oder einen sonstigen Kunststoff handeln. Bei dem Matrixmaterial kann es sich um das gleiche Matrixmaterial wie das des Kerns oder um ein anderes Matrixmaterial handeln.

Im Kern ist zumindest eine Mehrzahl der Fasern vorzugsweise in axialer Richtung des Führungsdrahtes ausgerichtet.

Für die Fasern des inneren Schaftes können z.B. nicht leitfähige Fasermaterialien eingesetzt werden, z.B. solche aus Kunststoff und/oder anorganische Materialien. Nicht leitfähige Materialien sind insbesondere vorteilhaft beim Einsatz im MRT. Geeignete Kunststoffe für die Fasern könne z.B. sein: Glas, Nylon (Polyamide), Polyester, PEEK, Polyacryl, Ultrahochmolekulargewichtiges Polyethylen (Ultra-High Molecular Weight Polyethylen; UHMWPE), Flüssigkristallpolymere (LCP), Aramide. Es können auch polymere optische Fasern (POF) eingesetzt werden. Diese Fasermaterialien können im Falle einer Struktur des inneren Schaftes aus Kern und Hüllschicht(en) sowohl für den Kern als auch für die mindestens eine Hüllschicht eingesetzt werden. Vorzugsweise werden Glasfasern im Kern und ein Aramid in der Hüllschicht des inneren Schaftes eingesetzt.

Der erfindungsgemäße Führungsdraht kann vorzugsweise mit dem erfindungsgemäßen Verfahren so ausgebildet werden, dass mindestens ein zur Markierung in einem bildgebenden Verfahren dienendes Markierungselement auf dem äußeren Umfang des inneren Schafts aufgebracht wird.

Auf diese Weise kann der Verlauf des Führungsdrahtes im Einsatz kontrolliert werden. Vorzugsweise ist das mindestens eine Markierungselement oder mindestens eines der Markierungselemente ein zur Markierung in der Magnetresonanztomographie geeignetes MRT-Markierungselement, das mindestens ein Markierungsmittel aufweist, welches bei Einsatz in einem Magnetresonanztomografen (MRT) aufgrund seiner Wechselwirkung mit den elektromagnetischen Wechselfeldern des MRT sichtbar ist. Das Markierungsmittel ist vorzugsweise ein solches, welches einen positiven Kontrast erzeugt, weiter vorzugsweise ein solches, welches die T1-Relaxationszeit und/oder die T2-Relaxationszeit reduziert. Hierzu gehören z.B. die Salze der Lanthaniden Gd ³⁺, Ho³⁺, Dy³⁺, Eu³⁺, die Komplexe einiger Übergangsmetalle, wie Fe³⁺, Mn²⁺, Mn³⁺ und Co³⁺. Derartige Agenzien ermöglichen die Sichtbarkeit des medizinischen Instruments in den gängigen MRT-Verfahren ohne besondere Maßnahmen. Weitere Agenzien, auch solche, die in anderen Bildgebungsverfahren, z.B. MPI (Magnetic Particle Imaging) oder in Verfahren unter Einsatz von Röntgenstrahlung, positive oder negative Kontraste erzeugen, können ebenfalls vorgesehen sein.

Als Markierungselement kann allein oder zusätzlich zu passiven Markierungselementen auch mindestens ein aktives Markierungselement eingesetzt werden. Aktiv bedeutet, dass das Markierungselement nicht nur passiv ein elektrisches oder magnetisches Feld und damit die Bildgebung beeinflusst, sondern aktiv ein elektrisches und/oder magnetisches Feld, insbesondere Wechselfeld, aussendet. Hierfür kann das aktive Markierungselement z.B. eine Spule, insbesondere eine Hochfrequenzspule (HF-Spule), aufweisen. Das aktive Markierungselement kann durch feine Leitungsdrähte durch den Führungsdraht hindurch mit Spannung versorgt werden. Es ist für den Betrieb in der MRT jedoch vorteilhaft, auf solche Drähte zu verzichten. Das aktive Markierungselement kann auch mittels Induktion vom Wechselfeld des Magnetresonanz-Tomographen oder einer sonstigen externen Wechselfeldquelle gespeist werden.

Der erfindungsgemäße Führungsdraht kann auch so ausgebildet sein, dass das mindestens eine Markierungselement außen auf die Hüllschicht aufgebracht ist. Das Markierungselement kann beispielsweise in einem Druckverfahren, also durch Aufdrucken, aufgebracht werden. Diese Vorgehensweise kann auch bei einem aktiven Markierungselement vorgesehen werden.

Vorzugsweise bildet das mindestens eine Markierungselement einen die Hüllschicht umfassenden Ring. Im Falle einer Mehrzahl von Markierungselementen können diese in axialer Richtung des Führungsdrahtes gesehen bestimmte Abstände zueinander aufweisen. Diese Abstände können gleichmäßig sein. Es ist aber auch denkbar, die Abstände zu variieren und als Kodierung für bestimmte Stellen des Führungsdrahtes zu nutzen. Zur Kodierung kann alternativ oder zusätzlich auch eine Variation der Länge der Markierungselemente in Längsrichtung des Führungsdrahtes eingesetzt werden. Weiter alternativ oder zusätzlich können unterschiedliche Markierungselemente unterschiedliche Konzentrationen des Markierungsmittels aufweisen.

Der erfindungsgemäße Führungsdraht kann auch so ausgebildet sein, dass die den inneren Schaft umgebende Schutzschicht ein Schutzmantel ist. Im Falle mehrerer Schutzschichten bildet der Schutzmantel die äußerste Schicht. Der Schutzmantel besteht vorzugsweise aus PTFE (Polytetrafluorethylen) oder weist zumindest PTFE auf, kann aber auch einen oder mehrere andere Kunststoffe aufweisen. Der Schutzmantel wird vorzugsweise unmittelbar auf den inneren Schaft oder, sofern der innere Schaft von weiteren inneren Schutzschichten umgeben sein sollte, auf die äußerste der inneren Schutzschichten aufgebracht, vorzugsweise aufgeschrumpft. Hierdurch erübrigt sich die Verwendung besonderer Kleber zur Fixierung des Schutzmantels. Außerdem ist ein Kleber zum Verschließen des Führungsdrahtes an den Enden nicht notwendig, da der Schutzmantel am distalen oder proximalen Ende bereits geschlossen sein kann oder das Material des Schutzmantels, insbesondere im Falle des PTFE, an einem oder beiden Enden verschweißt werden kann. Durch Temperatureinwirkung kann zudem eine stoffschlüssige Verbindung zwischen Schutzmantel und darunter liegender Schicht, insbesondere der Hüllschicht, erzeugt werden.

Des Weiteren kann es vorteilhaft sein, das erfindungsgemäße Verfahren so auszuführen, dass das mit dem Schutzmantel versehene Drahtendstück vor dem Aufschrumpfen des Schutzmantels in eine die Form des Drahtendstückes vorgebende Formfixiervorrichtung fixiert und bei fixiertem Drahtendstück der Schutzmantel aufgeschrumpft wird. Durch den Aufschrumpfprozess bleibt das Drahtendstück in seiner vorgegebenen Form stabil. Bevorzugt wird dem Drahtendstück in der Formfixiervorrichtung eine gekrümmte Form gegeben. Die Formfixiervorrichtung kann z.B. eine Nut mit dem gewünschten Verlauf aufweisen, in die das Drahtendstück vor dem Aufschrumpfen des Schutzmantels eingelegt wird. Die Nut kann mit einem Deckel verschlossen werden. Alternativ zu einer Nut kann die Formfixiervorrichtung eine veränderbare Formvorgabe aufweisen, z.B. durch verschiebbare oder steckbare Begrenzungselemente, die beispielsweise pinförmig sein können.

Erfindungsgemäß wird der Führungsdraht so ausgebildet bzw. das erfindungsgemäße Verfahren so ausgeführt, dass der Führungsdraht ein freies proximales Drahtendstück aufweist, welches sich an dem vom distalen Drahtendstück gegenüberliegenden Ende am Drahthauptstück anschließt. Ein freies proximales Drahtendstück ermöglicht es einem Benutzer, nach Wahl auch das proximale Drahtendstück als Führungsspitze einzusetzen. Die Funktion des proximalen Drahtendstücks und die des distalen Drahtendstücks können somit getauscht werden.

Der Aufbau des freien proximalen Drahtendstückes und der des distalen Drahtendstückes können einander entsprechen oder auch unterschiedlich sein. Bei unterschiedlichem Aufbau können unterschiedliche Eigenschaften, z.B. unterschiedliche Flexibilität oder unterschiedliche Formen, gegeben sein, die im Einsatz genutzt werden können.

So kann es sinnvoll sein, allein im distalen Drahtendstück eine Vielzahl von Schwächungsstellen vorzusehen, so dass der Nutzer ein flexibleres und ein steiferes Drahtendstück zur Verfügung hat. Es können aber auch beide Drahtendstücke mit jeweils einer Vielzahl von Schwächungsstellen versehen sein.

Des Weiteren kann dem proximalen Drahtendstück oder dem distalen Drahtendstück oder beiden Drahtendstücken eine gebogene Form, vorzugsweise mit einem Biegewinkel von mindestens 45° und höchsten 90° aufgegeben werden.

Wie im distalen Drahtendstück kann auch das proximale Drahtendstück einen inneren, ein erstes Faserverbundmaterial aufweisenden Schaft und mindestens eine den inneren Schaft umhüllende Schutzschicht aufweisen. Auch das Drahthauptstück kann einen solchen inneren, ein erstes Faserverbundmaterial aufweisenden Schaft und mindestens eine den inneren Schaft umhüllende Schutzschicht aufweisen. Innerer Schaft und Schutzschicht sind vorzugsweise in den Drahtendstücken und dem Drahthauptstück einander in Material und - von den Schwächungsstellen abgesehen - im Aufbau zumindest im Wesentlichen übereinstimmend, so dass die Drahtendstücke zusammen mit dem Drahthauptstück einstückig mit durchgehendem Kern, durchgehender Hüllschicht und durchgehender Schutzschicht sind. Bei Bedarf können jedoch auch im Drahthauptstück Schwächungsstellen vorgesehen werden.

Schließlich kann das bereits oben erwähnte mindestens eine Markierungselement am distalen Drahtendstück, am proximalen Drahtendstück oder jeweils an beiden Drahtendstücken angebracht sein.

Es kann auch sinnvoll sein, mindestens ein Markierungselement allein im Drahthauptstück oder im Drahthauptstück und zusätzlich mindestens ein Markierungselement in einem oder beiden der Drahtendstücke vorzusehen.

Dabei können im distalen Drahtendstück, im proximalen Drahtendstück und/oder im Drahthauptstück das mindestens eine Markierungselement oder mindestens eines der Markierungselemente auf dem äußeren Umfang des inneren Schafts aufgebracht sein. Das mindestens eine Markierungselement oder mindestens eines der Markierungselemente kann jeweils auch ein zur Markierung in der Magnetresonanztomographie geeignetes MRT-Markierungselement sein, wobei vorzugsweise das mindestens eine MRT-Markierungselement oder mindestens eines der MRT-Markierungselemente ein aktives Markierungselement ist.

Im Folgenden werden bevorzugte Ausführungsformen des erfindungsgemäßen Führungsdrahtes sowie des erfindungsgemäßen Herstellungsverfahrens anhand von Figuren dargestellt.

Es zeigt schematisch
Fig. 1: den vorderen Bereich eines ersten Führungsdrahtes mit distalem Drahtendstück,
Fig. 2: einen zweiten Führungsdraht mit distalem und proximalem Drahtendstück und
Fig. 3: einen vergrößerten Ausschnitt aus dem proximalen Drahtendstück.

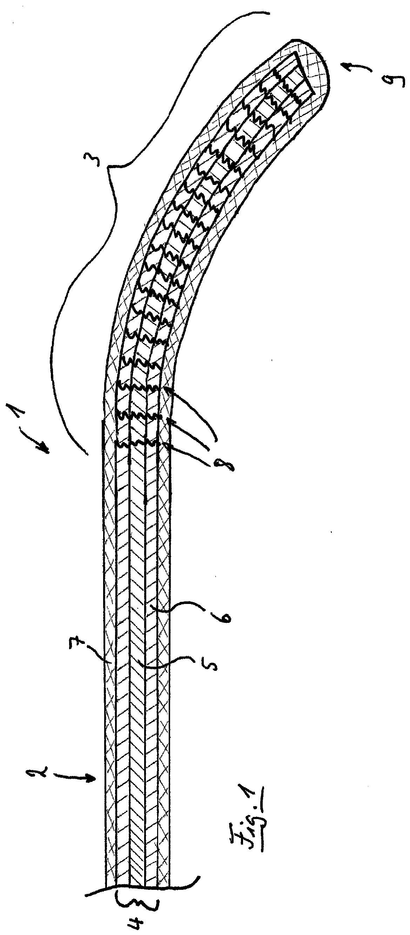

Fig. 1 zeigt den vorderen Bereich eines ersten Führungsdrahtes 1 mit einem Drahthauptstück 2 und einem sich nahtlos daran anschließenden distalen Drahtendstück 3. Ein erfindungsgemäß zugehöriges freies proximales Drahtendstück ist in Fig. 1 nicht dargestellt. Der erste Führungsdraht 1 weist einen inneren Schaft 4 auf, der aus einem Kern 5 und einer den Kern 5 umgebenden Hüllschicht 6 besteht. Auf den inneren Schaft 4 ist ein Schutzmantel 7, vorzugsweise aus PTFE, übergezogen und aufgeschrumpft. Die Größenverhältnisse sind in der Figur zur besseren zeichnerischen Darstellbarkeit nicht maßstäblich. Tatsächlich ist die Länge des Drahtendstückes 3 in der Größenordnung von vorzugsweise 30 mm bis 60 mm und der Durchmesser des ersten Führungsdrahtes 1 in der Größenordnung von weniger als 1 mm.

Der Kern 5 besteht aus einem Faserverbundmaterial, welches Glasfasern und als Kunststoffmatrix Epoxidharz aufweist. Die in der Fig. nicht dargestellten Glasfasern sind zumindest überwiegend in Längsrichtung des ersten Führungsdrahtes 1 ausgerichtet. Zur Herstellung des Kerns werden die Glasfasern, die vorzugsweise über die Länge des ersten Führungsdrahtes 1 durchgehend sind, in einem Pultrusionsverfahren mit der Kunststoffmatrix versehen.

Die Hüllschicht 6 besteht ebenfalls aus einem Faserverbundmaterial, wobei hier vorzugsweise Fasern aus einem Aramid eingesetzt werden. Die nicht dargestellten Fasern werden vorzugsweise in zwei unterschiedlichen Orientierungen helixartig um den Kern 5 gewickelt. Anschließend wird - ebenfalls im Pultrusionsverfahren - die Kunststoffmatrix für die Hüllschicht 6 angebracht. Bei der Kunststoffmatrix handelt es sich vorzugsweise ebenfalls um Epoxidharz. Die beiden Pultrusionsverfahren können während eines gemeinsamen Ziehvorganges durchgeführt werden.

Nach dem Fertigen des inneren Schafts 4 wird dieser im distalen Drahtendstück 3 durch mechanischen Eingriff mit einer Vielzahl von Schwächungsstellen 8 versehen, von denen in der Figur lediglich die vom distalen Ende 9 aus gesehen hinteren drei Schwächungsstellen 8 mit der Bezugszahl versehen sind. Die Schwächungsstellen 8 dienen dazu, die Biegesteifigkeit des Führungsdrahtes 1 im Drahtendstück 3 zu verringern. Die Schwächungsstellen 8 ersetzen somit das wesentlich aufwändigere aus dem Stand der Technik bekannte Reduzieren des Durchmessers des inneren Schaftes 4.

Um die Schwächungsstellen 8 zu erzeugen, kann der innere Schaft 4 mit seinem distalen Ende über eine hier nicht dargestellte mechanische Kante gelegt werden. Auf einen die mechanische Kante überragenden Teil des inneren Schaftes 4 wird eine Kraft mit einer Komponente senkrecht zur Längsrichtung des inneren Schaftes 4 auf den inneren Schaft 4 ausgeübt. Hierdurch kommt es bei ausreichender Kraft zu einer Bewegung des inneren Schaftes 4 mit einer Beuge- Knick- und/oder Brechbelastung, welche zu einer Rissbildung im inneren Schaft 4 führt. Da insbesondere die Glasfasern im Kern 5 bei der besagten Bewegung überwiegend nicht gebrochen werden, bleibt der innere Schaft 4 zusammenhängend und ein vollständiges Abbrechen eines Teils des inneren Schaftes 4 kann vermieden werden. Die Beuge-, Knick- und oder Brechbelastung führt vielmehr zu einem teilweisen Aufreißen der Kunststoffmatrix, wodurch die Biegesteifigkeit des inneren Schaftes 4 an der erzeugten Schwächungsstelle 8 deutlich reduziert wird.

Die mechanische Schwächung wird nun vielfach wiederholt, zum Beispiel indem der innere Schaft 4 weiter über die mechanische Kante hinausgeschoben wird, bis die angreifende Kraft die nächste Schwächungsstelle 8 erzeugt. In einem distalen Drahtendstück 3 von beispielsweise 40 mm Länge werden vorzugsweise zwanzig Schwächungsstellen 8 mit Abstand von etwa 2 mm angefertigt. Das distale Drahtendstück 3 kann aber auch z.B. eine Länge von 30 mm bis 60 mm aufweisen, wobei die Abstände zwischen den Schwächungsstellen vorzugsweise 1 bis 3 mm betragen. Der gesamte Vorgang kann dann mit einer geänderten Drehwinkelposition des inneren Schaftes 4 wiederholt werden. Hierfür wird beispielsweise der innere Schaft 4 nach dem ersten Durchgang des Einbringens einer Vielzahl von Schwächungsstellen 8 um etwa 90° relativ zur Richtung der angreifenden Kraft um die Längsachse gedreht und in einem zweiten Durchgang in entsprechender Weise behandelt.

Die angreifende Kraft kann zum Beispiel mittels der Gewichtskraft einer hier nicht dargestellten, am distalen Ende des inneren Schaftes 4 fixierten Masse erzeugt werden.

Nachdem der innere Schaft 4 mit der gewünschten Anzahl von Schwächungsstellen 8 versehen ist, wird über den inneren Schaft 4 der Schutzmantel 7 gezogen. Anschließend wird optional zumindest das distale Drahtendstück 3 in eine gewünschte Form gebracht und in einer hier nicht dargestellten Formfixiervorrichtung fixiert. Anschließend wird der Schutzmantel 7 bei geeigneter Temperaturführung auf die innere Struktur 4 aufgeschrumpft. Nach dem Abkühlen bleibt die Form des Drahtendstückes 3 aufgrund der stabilisierenden Wirkung des aufgeschrumpften Schutzmantels 7 auch nach dem Entfernen aus der Formfixiereinrichtung bestehen. Die aufgegebene Form ist zumindest in einem Teilstück vorzugsweise eine Bogenform.

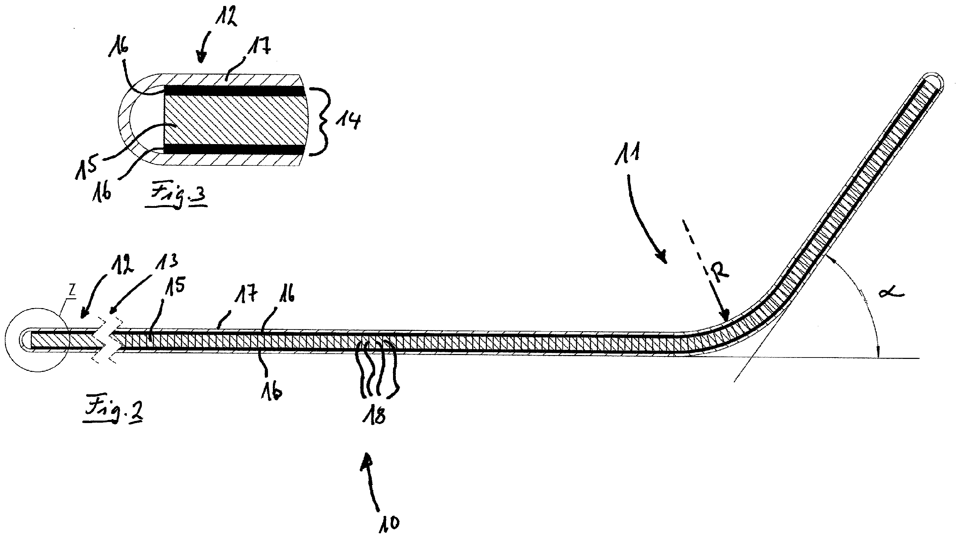

Fig. 2 zeigt einen zweiten Führungsdraht 10 mit einem distalen Drahtendstück 11 und einem proximalen Drahtendstück 12. Ein Drahthauptstück ist hier nicht dargestellt und fällt in die Unterbrechung 13 der Darstellung des zweiten Führungsdrahtes 10. Die Drahtendstücke 11 und 12 weisen - abgesehen von später erläuterten Schwächungsstellen - im Wesentlichen einen übereinstimmenden Aufbau auf. Ein in Fig. 2 mit "Z" markierter Bereich des proximalen Drahtendstücks 12 ist in Fig. 3 vergrößert dargestellt. In der Vergrößerung sind ein Kern 15 des zweiten Führungsdrahtes 10, eine den Kern 15 umgebende Hüllschicht 16 und eine Schutzschicht in Form eines Schutzmantels 17 zu erkennen. Kern 15 und Hüllschicht 16 bilden zusammen den inneren Schaft 14. Somit stimmt der zweite Führungsdraht 10 im Aufbau mit dem Drahthauptstück 2 und dem distalen Drahtendstück 3 des ersten Führungsdrahts 1 gemäß Fig. 1 überein.

Das distale Drahtendstück 11 weist im inneren Schaft 14 eine Vielzahl von Schwächungsstellen 18 auf, welche durch zur Längsachse des zweiten Führungsdrahtes 10 senkrechte Striche symbolisiert und von denen lediglich vier mit Bezugszeichen gekennzeichnet sind. Die Schwächungsstellen 18 verteilen sich über die gesamte Länge des dargestellten distalen Drahtendstücks 11 und haben eine erhöhte Flexibilität des distalen Drahtendstückes 11 zur Folge. Die Herstellung der Schwächungsstellen kann auf die gleiche Weise erfolgen, wie sie bereits zu Fig. 1 beschreiben ist, oder auf geeignete andere Weise.

Das proximale Drahtendstück 12 ist im Ausführungsbeispiel der Fig. 2 hingegen ohne Schwächungsstellen ausgebildet. Ein Nutzer kann sich entscheiden, ob er das distale Drahtendstück 11 oder das proximale Drahtendstück 12 als Spitze des zweiten Führungsdrahtes 10 für die Führung eines anderen Instruments, z.B. eines hier nicht dargestellten Katheters, nutzt.

Das distale Drahtendstück 11 ist in einem Teilstück 19 gebogen, wobei der Biegewinkel α etwa 63° beträgt. Die Biegung kann gleichmäßig mit einem konstanten Biegeradius R oder ungleichmäßig mit sich änderndem Biegeradius R sein. Auch die Herstellung der Biegung und/oder das Aufbringen des Schutzmantels 17 kann auf die gleiche Weise erfolgen, wie sie bzw. es bereits zu Fig. 1 beschreiben ist, oder auf geeignete andere Weise.

### Bezugszeichenliste

- 1: Erster Führungsdraht
- 2: Drahthauptstück
- 3: Drahtendstück
- 4: Innerer Schaft
- 5: Kern
- 6: Hüllschicht
- 7: Schutzmantel
- 8: Schwächungsstelle
- 9: Distales Ende
- 10: Zweiter Führungsdraht
- 11: Distales Drahtendstück
- 12: Proximales Drahtendstück
- 13: Unterbrechung
- 14: Innerer Schaft
- 15: Kern
- 16: Hüllschicht
- 17: Schutzmantel
- 18: Schwächungsstellen
- 19: Teilstück
- α: Biegewinkel
- R: Biegeradius

## Patentansprüche

1. Führungsdraht für minimalinvasive Eingriffe mit einem an einem Drahthauptstück (2) anschließenden distalen Drahtendstück (3, 11), wobei
a) der Führungsdraht (1, 10) zumindest in dem distalen Drahtendstück (3, 11) einen inneren Schaft (4, 14) und mindestens eine den inneren Schaft (4, 14) umhüllende Schutzschicht aufweist,
b) der innere Schaft (4, 14) ein erstes Faserverbundmaterial aufweist, und
c) zumindest im distalen Drahtendstück (3, 11) der innere Schaft (4, 14) eine Vielzahl von Schwächungsstellen (8, 18) aufweist, welche durch mechanische Eingriffe erzeugt sind,
**dadurch gekennzeichnet, dass**
d) der innere Schaft (4, 14) einen Kern (5, 15) und mindestens eine den Kern (5, 15) umgebende Hüllschicht (6, 16) aufweist,
e) der Kern (5, 15) das erste Faserverbundmaterial aufweist, wobei das erste Faserverbundmaterial Glasfasern aufweist,
f) die mindestens eine Hüllschicht (6, 16) oder mindestens eine der Hüllschichten (6, 16) ein zweites Faserverbundmaterial aufweist, wobei das zweite Faserverbundmaterial Aramidfasern aufweist, welche von einem Matrixmaterial aus Kunstharz umhüllt sind,
g) die Schwächungsstellen (8, 18) durch Knickbelastung, Biegebelastung und/oder Brechbelastung erzeugt sind und
h) ein freies proximales Drahtendstück (12) gegeben ist, welches sich an dem vom distalen Drahtendstück (11) gegenüberliegenden Ende am Drahthauptstück (2) anschließt.

2. Führungsdraht nach Anspruch 1, **dadurch gekennzeichnet, dass** mindestens eine Teilanzahl von Fasern des zweiten Faserverbundmaterials helixartig um den Umfang des Kerns (5, 15) geführt sind, wobei vorzugsweise die mindestens eine Teilanzahl der Fasern des zweiten Faserverbundmaterials in zwei entgegengesetzten unterschiedlichen Helixorientierungen um den Umfang des Kerns (5, 15) geführt sind.

3. Führungsdraht nach einem der vorherigen Ansprüche, **dadurch gekennzeichnet, dass** die Schutzschicht oder im Falle mehrerer Schutzschichten die äußerste Schutzschicht ein aus PTFE bestehender oder zumindest PTFE enthaltender Schutzmantel (7, 17) ist.

4. Führungsdraht nach einem der vorherigen Ansprüche, **gekennzeichnet durch** mindestens ein zur Markierung in einem bildgebenden Verfahren geeignetes Markierungselement, wobei vorzugsweise das Markierungselement oder mindestens eines der Markierungselemente auf dem äußeren Umfang des inneren Schafts (4, 14) aufgebracht ist.

5. Führungsdraht nach Anspruch 4, **dadurch gekennzeichnet, dass** das mindestens eine Markierungselement oder mindestens eines der Markierungselemente ein zur Markierung in der Magnetresonanztomographie geeignetes MRT-Markierungselement ist, wobei vorzugsweise das mindestens eine MRT-Markierungselement oder mindestens eines der MRT-Markierungselemente ein aktives Markierungselement ist.

6. Führungsdraht nach einem der vorhergehenden Ansprüche, **dadurch gekennzeichnet, dass** der Führungsdraht (1, 10) auch in dem proximalen Drahtendstück (12) einen ein erstes Faserverbundmaterial aufweisenden inneren Schaft (4, 14) und mindestens eine den inneren Schaft (4, 14) umhüllende Schutzschicht aufweist.

7. Führungsdraht nach Anspruch 6, **dadurch gekennzeichnet, dass** im proximalen Drahtendstück (12) der innere Schaft (4, 14) eine Vielzahl von Schwächungsstellen (8, 18) aufweist, welche durch mechanische Eingriffe, bevorzugt durch Knickbelastung, Biegebelastung und/oder Brechbelastung, erzeugt sind.

8. Führungsdraht nach einem der vorhergehenden Ansprüche, **gekennzeichnet durch** mindestens ein im proximalen Drahtendstück (12) angeordnetes Markierungselement gemäß einem der Ansprüche 4 oder 5.

9. Führungsdraht nach einem der vorherigen Ansprüche, **dadurch gekennzeichnet, dass** dem distalen Drahtendstück (3, 11) und/oder dem proximalen Drahtendstück (12) eine gebogene Form aufgegeben ist.

10. Führungsdraht nach Anspruch 9, **dadurch gekennzeichnet, dass** die gebogene Form einen Biegewinkel (α) von mindestens 45° und höchstens 90° aufweist.

11. Verfahren zur Herstellung eines Führungsdrahtes für minimalinvasive Eingriffe mit einem an einem Drahthauptstück (2) anschließenden distalen Drahtendstück (3, 11), wobei zumindest im distalen Drahtendstück (3, 11) ein ein erstes Faserverbundmaterial aufweisender inneren Schaft (4, 14) vorhanden ist,
bei dem zumindest im distalen Drahtendstück (3, 11) der innere Schaft (4, 14) durch mechanische Eingriffe mit einer Vielzahl von Schwächungsstellen (8, 18) versehen wird,
**dadurch gekennzeichnet, dass**
die Schwächungsstellen (8, 18) durch Knickbelastung, Biegebelastung und/oder Brechbelastung erzeugt werden und
am Führungsdraht (1, 10) ein freies proximales Drahtendstück (12) hergestellt wird, welches sich an dem vom distalen Drahtendstück (11) gegenüberliegenden Ende am Drahthauptstück (2) anschließt.

12. Verfahren nach Anspruch 11, **dadurch gekennzeichnet, dass** auch in dem proximalen Drahtendstück (12) ein innerer ein erstes Faserverbundmaterial aufweisender Schaft (4, 14) und mindestens eine den inneren Schaft (4, 14) umhüllende Schutzschicht angeordnet wird.

13. Verfahren nach Anspruch 12, **dadurch gekennzeichnet, dass** im proximalen Drahtendstück (12) im inneren Schaft (4, 14) eine Vielzahl von Schwächungsstellen (8, 18) durch mechanische Eingriffe, bevorzugt durch Knickbelastung, Biegebelastung und/oder Brechbelastung erzeugt werden.

14. Verfahren nach einem der Ansprüche 11 bis 13, **dadurch gekennzeichnet, dass** für die mechanischen Eingriffe der innere Schaft (4, 14) über mindestens eine mechanische Kante gelegt und mit einer quer zur Längsachse des ungeschwächten Drahtendstücks wirkenden Kraft beaufschlagt wird, wobei vorzugsweise die Kraftbeaufschlagung entlang des inneren Schaftes in Abständen von 1 mm bis 3 mm durchgeführt wird.

15. Verfahren nach einem der Ansprüche 11 bis 14, **dadurch gekennzeichnet, dass** die mechanischen Eingriffe in mindestens zwei unterschiedlichen, auf eine Rotation um die Längsachse des inneren Schaftes (4, 14) bezogenen Drehwinkelpositionen des inneren Schafts (4, 14) durchgeführt werden, vorzugsweise in um 90° +/- 10° voneinander abweichenden Drehwinkelpositionen.

16. Verfahren nach einem der Ansprüche 11 bis 15, **dadurch gekennzeichnet, dass** zur Erzeugung des inneren Schafts (4, 14) ein Kern (5, 15) aus einem ersten Faserverbundmaterial mit mindestens einer Hüllschicht (6, 16) aus einem, bevorzugt vom ersten Faserverbundmaterial verschiedenen, zweiten Faserverbundmaterial umgeben wird, wobei Fasern des zweiten Faserverbundmaterials mit entgegengesetzten Helixorientierungen um den Kern (5, 15) geführt werden.

17. Verfahren nach einem der Ansprüche 11 bis 16, **dadurch gekennzeichnet, dass** mindestens ein zur Markierung in einem bildgebenden Verfahren dienendes Markierungselement auf dem äußeren Umfang des inneren Schafts (4, 14) aufgebracht wird.

18. Verfahren nach Anspruch 17, **dadurch gekennzeichnet, dass** das mindestens eine Markierungselement zumindest auch am distalen Drahtendstück (3, 11) und/oder am proximalen Drahtendstück (12) angebracht wird

19. Verfahren nach einem der Ansprüche 11 bis 18, **dadurch gekennzeichnet, dass** der innere Schaft (4, 14) nach dem mechanischen Eingriff mit mindestens einer Schutzschicht umgeben wird, wobei vorzugsweise als Schutzschicht oder im Falle mehrerer Schutzschichten als äußerste Schutzschicht ein Schutzmantel (7, 17), vorzugsweise aus PTFE, aufgeschrumpft wird.

20. Verfahren nach einem der Ansprüche 11 bis 19, **dadurch gekennzeichnet, dass** dem distalen Drahtendstück (3, 11) und/oder dem proximalen Drahtendstück (12) eine gebogene Form aufgegeben wird.

21. Verfahren nach Anspruch 20, **dadurch gekennzeichnet, dass** der gebogenen Form ein Biegewinkel (α) von mindestens 45° und höchstens 90° aufgegeben wird.

## Claims

1. Guidewire for minimally invasive interventions, with a distal wire endpiece (3, 11) adjoining a wire main piece (2), wherein
a) the guidewire (1, 10) has, at least in the distal wire endpiece (3, 11), an inner shaft (4, 14) and at least one protective layer enveloping the inner shaft (4, 14),
b) the inner shaft (4, 14) has a first fiber composite material, and
c) at least in the distal wire endpiece (3, 11), the inner shaft (4, 14) has a multiplicity of weakened sites (8, 18), which are generated by mechanical interventions,
**characterized in that**
d) the inner shaft (4, 14) has a core (5, 15) and at least one envelope layer (6, 16) surrounding the core (5, 15),
e) the core (5, 15) has the first fiber composite material, wherein the first fiber composite material has glass fibers,
f) the at least one envelope layer (6, 16) or at least one of the envelope layers (6, 16) has a second fiber composite material, wherein the second fiber composite material has aramid fibers which are encased by a matrix material of synthetic resin,
g) the weakened sites (8, 18) are generated by buckling loads, bending loads and/or breaking loads, and
h) a free proximal wire endpiece (12) is given which adjoins the end of the wire main piece (2) opposite the distal wire endpiece (11).

2. Guidewire according to claim 1, **characterized in that** at least one subset of the fibers of the second fiber composite material is guided helically about the circumference of the core (5, 15), wherein the at least one subset of fibers of the second fiber composite material is preferably guided in two oppositely directed, different helical orientations about the circumference of the core (5, 15).

3. Guidewire according to one of the preceding claims, **characterized in that** the protective layer or, in the case of several protective layers, the outermost protective layer is a protective jacket (7, 17) consisting of PTFE or at least containing PTFE.

4. Guidewire according to one of the preceding claims, **characterized by** at least one marking element suitable for marking in an imaging method, wherein the marking element or at least one of the marking elements is preferably applied to the outer circumference of the inner shaft (4, 14).

5. Guidewire according to claim 4, **characterized in that** the at least one marking element or at least one of the marking elements is an MRT marking element suitable for marking in magnetic resonance tomography, wherein the at least one MRT marking element or at least one of the MRT marking elements is preferably an active marking element.

6. Guidewire according to one of the preceding claims, **characterized in that** the guidewire (1, 10) also has, in the proximal wire endpiece (12), an inner shaft (4, 14) having a first fiber composite material, and at least one protective layer enveloping the.

7. Guidewire according to claim 6, **characterized in that**, in the proximal wire endpiece (12), the inner shaft (4, 14) has a multiplicity of weakened sites (8, 18) which are generated by mechanical interventions, preferably by buckling loads, bending loads and/or breaking loads.

8. Guidewire, **characterized by** at least one marking element, as per either of claims 4 and 5, arranged in the proximal wire endpiece (12).

9. Guidewire according to one of the preceding claims, **characterized in that** a bent shape is conferred on the distal wire endpiece (3, 11) and/or to the proximal wire endpiece (12).

10. Guidewire according to claim 9, **characterized in that** the bent shape has a bending angle (α) of at least 45° and at most 90°.

11. Method for producing a guidewire for minimally invasive interventions, with a distal wire endpiece (3, 11) adjoining a wire main piece (2), wherein, at least in the distal wire endpiece (3), an inner shaft (4, 14) having a first fiber composite material is present,
and, at least in the distal wire endpiece (3, 11), the inner shaft (4,14) is provided with a multiplicity of weakened sites (8, 18) generated by mechanical interventions,
**characterized in that**
the weakened sites (8, 18) are generated by buckling loads, bending loads and/or breaking loads and
on the guidewire (1, 10), a free proximal wire endpiece (12) is produced which adjoins the end of the wire main piece (2) opposite the distal wire endpiece (11).

12. Method according to claim 11, **characterized in that** an inner shaft (4, 14) having a first fiber composite material is also arranged in the proximal wire endpiece (12), and moreover at least one protective layer enveloping the inner shaft (4, 14).

13. Method according to claim 12, **characterized in that** in the inner shaft (4, 14) in the proximal wire endpiece (12), a multiplicity of weakened sites (8, 18) is generated by mechanical interventions, preferably by buckling loads, bending loads and/or breaking loads.

14. Method according to one of the claims 11 to 13, **characterized in that** for the mechanical interventions, the inner shaft (4, 14) is placed over at least one mechanical edge and is subjected to a force acting transversely with respect to the longitudinal axis of the unweakened wire endpiece, wherein the force is preferably applied along the inner shaft at intervals of 1 mm to 3 mm.

15. Method according to one of the claims 11 to 14, **characterized in that** the mechanical interventions are carried out in at least two different rotation angle positions of the inner shaft (4, 14) relative to a rotation about the longitudinal axis of the inner shaft (4, 14), preferably in rotation angle positions deviating from each other by 90° +/-10°.

16. Method according to one of the claims 11 to 15, **characterized in that** in order to generate the inner shaft (4, 14), a core (5, 15) made of a first fiber composite material is surrounded by at least one envelope layer (6, 16) made of a second fiber composite material preferably different than the first fiber composite material, wherein fibers of the second fiber composite material are guided around the core (5, 15) with oppositely directed helical orientations.

17. Method according to one of the claims 11 to 16, **characterized in that** at least one marking element serving for marking purposes in an imaging method is applied to the outer circumference of the inner shaft (4, 14).

18. Method according to claim 17, **characterized in that** the at least one marking element is also applied at least to the distal wire endpiece (3, 11) and/or to the proximal wire endpiece (12).

19. Method according to one of the claims 11 to 18, **characterized in that** the inner shaft (4, 14) is surrounded by at least one protective layer after the mechanical intervention, wherein a protective jacket (7, 17), preferably of PTFE, is preferably shrink-fitted as the protective layer or, in the case of several protective layers, as the outermost protective layer.

20. Method according to one of the claims 11 to 19, **characterized in that** a bent shape is conferred on the distal wire endpiece (3, 11) and/or on the proximal wire endpiece (12).

21. Method according to claim 20, **characterized in that** the bent shape has a bending angle (α) of at least 45° and at most 90°.

## Revendications

1. Fil de guidage pour des interventions à invasion minimale avec une pièce d'extrémité distale (3, 11) du fil se raccordant à une pièce principale (2) du fil, dans lequel
a) le fil de guidage (1, 10) présente au moins dans la pièce d'extrémité distale (3, 11) du fil une tige intérieure (4, 14) et au moins une couche de protection enveloppant la tige intérieure (4, 14),
b) la tige intérieure (4, 14) présente un premier matériau composite fibreux, et
c) dans la pièce d'extrémité (3, 11) du fil la tige intérieure (4, 14) présente une multiplicité de points faibles (8, 18), qui sont produits par des interventions mécaniques,
**caractérisé en ce que**
d) la tige intérieure (4, 14) présente une âme (5, 15) et au moins une couche d'enveloppe (6, 16) entourant l'âme (5, 15),
e) l'âme (5, 15) présente le premier matériau composite fibreux, dans lequel le premier matériau composite fibreux présente des fibres de verre;
f) ladite au moins une couche d'enveloppe (6, 16) ou au moins une des couches d'enveloppe (6, 16) présente un deuxième matériau composite fibreux, dans lequel le deuxième matériau composite fibreux présente des fibres d'aramide, qui sont enveloppées par un matériau de matrice en résine synthétique,
g) les points faibles (8, 18) sont produits par une charge de flambage, une charge de flexion et/ou une charge de rupture et
h) une pièce d'extrémité proximale (12) libre du fil est donnée, qui se raccorde à la pièce principale de fil (2) à l'extrémité opposée à la pièce d'extrémité distale (11) du fil.

2. Fil de guidage selon la revendication 1, **caractérisé en ce qu'**au moins un nombre partiel de fibres du deuxième matériau composite fibreux sont conduites en forme d'hélice autour de la périphérie de l'âme (5, 15), dans lequel, de préférence, ledit au moins un nombre partiel de fibres du deuxième matériau composite fibreux est conduit dans deux orientations hélicoïdales différentes opposées autour de la périphérie de l'âme (5, 15).

3. Fil de guidage selon l'une quelconque des revendications précédentes, **caractérisé en ce que** la couche de protection ou dans le cas de plusieurs couches de protection la couche de protection la plus extérieure est une gaine de protection (7, 17) composée de PTFE ou au moins contenant du PTFE.

4. Fil de guidage selon l'une quelconque des revendications précédentes, **caractérisé par** au moins un élément de marquage convenant pour un marquage dans un procédé d'imagerie, dans lequel l'élément de marquage ou au moins un des éléments de marquage est de préférence apposé sur la périphérie extérieure de la tige intérieure (4, 14).

5. Fil de guidage selon la revendication 4, **caractérisé en ce que** ledit au moins un élément de marquage ou au moins un des éléments de marquage est un élément de marquage MRT convenant pour le marquage dans la tomographie par résonance magnétique, dans lequel ledit au moins un élément de marquage MRT ou au moins un des éléments de marquage MRT est de préférence un élément de marquage actif.

6. Fil de guidage selon l'une quelconque des revendications précédentes, **caractérisé en ce que** le fil de guidage (1, 10) présente également dans la pièce d'extrémité proximale (12) du fil une tige intérieure (4, 14) présentant un premier matériau composite fibreux et au moins une couche de protection enveloppant la tige intérieure (4, 14).

7. Fil de guidage selon la revendication 6, **caractérisé en ce que** dans la pièce d'extrémité proximale (12) du fil, la tige intérieure (4, 14) présente une pluralité de points faibles (8, 18), qui sont produits par des interventions mécaniques, de préférence par une charge de flambage, une charge de flexion et/ou une charge de rupture.

8. Fil de guidage selon l'une quelconque des revendications précédentes, **caractérisé par** au moins un élément de marquage selon l'une des revendications 4 ou 5, disposé dans la pièce d'extrémité proximale (12) du fil.

9. Fil de guidage selon l'une quelconque des revendications précédentes, **caractérisé en ce qu'**une forme courbée est appliquée à la pièce d'extrémité distale (3, 11) du fil et/ou à la pièce d'extrémité proximale (12) du fil.

10. Fil de guidage selon la revendication 9, **caractérisé en ce que** la forme courbée présente un angle de courbure (α) d'au moins 45° et d'au plus 90°.

11. Procédé de production d'un fil de guidage pour des interventions à invasion minimale avec une pièce d'extrémité distale (3, 11) du fil se raccordant à une pièce principale (2) du fil, dans lequel il se trouve au moins dans la pièce d'extrémité distale (3, 11) du fil une tige intérieure (4, 14) présentant un premier matériau composite fibreux,
dans lequel la tige intérieure (4, 14) est munie au moins dans la pièce d'extrémité distal (3, 11) du fil d'une multiplicité de points faibles (8, 18) par des interventions mécaniques,
**caractérisé en ce que** l'on produit les points faibles (8, 18) par une charge de flambage, une charge de flexion et/ou une charge de rupture et
on réalise sur le fil de guidage (1, 10) une pièce d'extrémité proximale (12) libre du fil qui se raccorde à la pièce principale (2) du fil à l'extrémité opposée à la pièce d'extrémité distale (11) du fil.

12. Procédé selon la revendication 11, **caractérisé en ce que** l'on dispose également dans la pièce d'extrémité proximale (12) du fil une tige intérieure (4, 14) présentant un premier matériau composite fibreux et au moins une couche de protection enveloppant la tige intérieure (4, 14).

13. Procédé selon la revendication 12, **caractérisé en ce qu'**une pluralité de points faibles (8, 18) sont générés dans la pièce d'extrémité proximale (12) du fil dans la tige intérieure (4, 14) par des interventions mécaniques, de préférence par une charge de flambage, une charge de flexion et/ou une charge de rupture.

14. Procédé selon une des revendications 11 à 13, **caractérisé en ce que** pour les interventions mécaniques on pose la tige intérieure (4, 14) sur au moins une arête mécanique et on la soumet à une force agissant transversalement à l'axe longitudinal de la pièce d'extrémité de fil non affaiblie, dans lequel on effectue l'application d'une force de préférence le long de la tige intérieure à des distances de 1 mm à 3 mm.

15. Procédé selon l'une quelconque des revendications 11 à 14, **caractérisé en ce que** l'on effectue les interventions mécaniques en au moins deux positions d'angle de rotation différentes de la tige intérieure (4, 14) par rapport à une rotation autour de l'axe longitudinal de la tige intérieure (4, 14), de préférence dans des positions d'angle de rotation espacées en rotation de 90° ± 10° l'une de l'autre.

16. Procédé selon l'une quelconque des revendications 11 à 15, **caractérisé en ce que** pour la production de la tige intérieure (4, 14) on entoure une âme (5, 15) en un premier matériau composite fibreux avec au moins une couche d'enveloppe (6, 16) en un deuxième matériau composite fibreux, de préférence différent du premier matériau composite fibreux, dans lequel on conduit des fibres du deuxième matériau composite fibreux avec des orientations hélicoïdales opposées autour de l'âme (5, 15).

17. Procédé selon l'une quelconque des revendications 11 à 16, **caractérisé en ce que** l'on appose au moins un élément de marquage servant pour le marquage dans un procédé d'imagerie sur la périphérie extérieure de la tige intérieure (4, 14).

18. Procédé selon la revendication 17, **caractérisé en ce que** ledit au moins un élément de marquage est appliqué au moins également sur la pièce d'extrémité distale (3, 11) du fil et/ou sur la pièce d'extrémité proximale (12) du fil.

19. Procédé selon l'une quelconque des revendications 11 à 18, **caractérisé en ce que** l'on entoure la tige intérieure (4, 14) après l'intervention mécanique avec au moins une couche de protection, dans lequel on rétracte de préférence une gaine de protection (7, 17), de préférence en PTFE, comme couche de protection ou dans le cas de plusieurs couches de protection comme la couche de protection la plus extérieure.

20. Procédé selon l'une quelconque des revendications 11 à 19, **caractérisé en ce qu'**une forme courbée est appliquée à la pièce d'extrémité distale (3, 11) du fil et/ou à la pièce d'extrémité proximale (12) du fil

21. Procédé selon la revendication 20, **caractérisé en ce qu'**un angle de courbure (α) d'au moins 45° et d'au plus 90° est appliqué à la forme courbée.
